Europäisches Patentamt

European Patent Office

Office européen des brevets

(19)

(11) EP 0 658 352 B1

(12) **FASCICULE DE BREVET EUROPEEN**

(45) Date de publication et mention
de la délivrance du brevet:
**15.10.1997 Bulletin 1997/42**

(51) Int. Cl.⁶: **A61M 1/16**

(21) Numéro de dépôt: **94420340.5**

(22) Date de dépôt: **06.12.1994**

(54) **Procédé de détermination d'un paramètre significatif du progrès d'un traitement extracorporel de sang**

Verfahren zum Bestimmen eines Fortschrittbestimmenden Parameters bei einer extrakorporalen Blutbehandlung

Procedure for determining a parameter indicating the progress of an extracorporeal blood treatment

(84) Etats contractants désignés:
**BE CH DE ES FR GB IT LI NL**

(30) Priorité: **17.12.1993 FR 9315527**
**23.03.1994 FR 9403710**

(43) Date de publication de la demande:
**21.06.1995 Bulletin 1995/25**

(73) Titulaire: **HOSPAL AG**
**4008 Basel (CH)**

(72) Inventeurs:
• **Goux, Nicolas**
**F-69290 Craponne (FR)**
• **Bene, Bernard**
**F-69540 Irigny (FR)**

(74) Mandataire: **Lejeune, Daniel**
**Hospal Service Brevets,**
**B.P. 21**
**69881 Meyzieu Cédex (FR)**

(56) Documents cités:
**EP-A- 0 291 421        EP-A- 0 428 927**
**EP-A- 0 547 025**

**Description**

L'invention concerne un procédé de détermination d'un paramètre significatif du progrès d'un traitement extracorporel de sang, en particulier d'un traitement d'épuration ayant pour but de pallier l'insuffisance rénale, comme l'hémodialyse ou l'hémodiafiltration.

Pour mémoire, l'hémodialyse consiste à faire circuler, de part et d'autre de la membrane semi-perméable d'un échangeur, le sang d'un patient et un liquide de traitement sensiblement isotonique au sang, de sorte que, lors du transfert diffusif qui s'établit au travers de la membrane pour les substances ayant des concentrations différentes de part et d'autre de la membrane, les impuretés du sang (urée, créatinine, etc.) migrent du sang vers le liquide de traitement. La concentration électrolytique du liquide de traitement est aussi généralement choisie pour corriger la concentration électrolytique du sang du patient.

Dans le traitement par hémodiafiltration, au transfert diffusif obtenu par dialyse s'ajoute un transfert convectif par ultrafiltration résultant d'une différence de pression positive créée entre le coté sang et le coté liquide de traitement de la membrane.

Il est du plus grand intérêt de pouvoir déterminer, tout au long d'une séance de traitement, un ou plusieurs paramètres significatifs du progrès du traitement afin de pouvoir, le cas échéant, modifier les conditions du traitement telles qu'elles ont été fixées initialement en vue d'un objectif thérapeutique déterminé.

Les paramètres dont la connaissance permet de suivre le progrès du traitement, c'est-à-dire aussi d'apprécier l'adéquation des conditions de traitement fixées initialement à l'objectif thérapeutique, sont, en particulier, la concentration du sang en un soluté donné (sodium par exemple), ou la dialysance réelle ou la clairance réelle de l'échangeur pour tel ou tel soluté (la dialysance et la clairance représentant le rendement épuratif de l'échangeur) ou la dose de dialyse administrée après un temps t de traitement, laquelle, d'après les travaux de Sargent et Gotch, peut être assimilée au rapport sans dimension $Kt/V$, où $K$ est la clairance réelle pour l'urée, $t$ le temps de traitement écoulé, et $V$ le volume de distribution de l'urée, c'est-à-dire le volume d'eau total du patient (Gotch FA, Sargent SA. A mechanistic analysis of the National Cooperative Dialysis Study (NCDS). Kidney int 1985 ; 28 : 526-34).

Ces paramètres posent tous le même problème pour leur détermination, qui est de nécessiter la connaissance précise d'une caractéristique physique ou chimique du sang, alors que cette caractéristique ne peut pas, dans la pratique, être obtenue par mesure directe sur un échantillon pour des raisons thérapeutiques, prophylactiques et pécuniaires : d'une part, il est exclu de prélever sur un patient, souvent anémié, les échantillons multiples qui seraient nécessaires pour suivre l'efficacité du traitement au cours de son déroulement ; d'autre part, compte tenu des risques liés à la manipulation d'échantillons de sang éventuellement contaminé, la tendance générale est à éviter de telles manipulations ; enfin, l'analyse d'un échantillon de sang en laboratoire est à la fois coûteuse et relativement longue, ce qui est incompatible avec l'objectif recherché.

Le document EP 0 547 025 décrit un procédé pour la détermination in vivo des paramètres de l'hémodialyse ne nécessitant pas d'effectuer des mesures sur le sang. Selon ce procédé, dont la mise en oeuvre requiert des moyens pour régler la concentration ionique du liquide de dialyse et des moyens pour mesurer la concentration en sodium du liquide de dialyse ou sa conductivité, le transfert électrolytique du liquide de dialyse est mesuré à deux concentrations différentes prédéterminées du liquide de dialyse et la dialysance en est déduite.

Ce procédé nécessite d'exposer le patient à un liquide de dialyse différant sensiblement du liquide de dialyse prescrit pendant le temps nécessaire à la stabilisation de la concentration du liquide de dialyse en aval de l'échangeur, faute de quoi la mesure porte sur un liquide de dialyse de concentration intermédiaire, et tous les calculs faits ultérieurement à partir de cette mesure sont erronés.

Un but de l'invention est de concevoir un procédé du type mentionné ci-dessus grâce auquel les paramètres représentatifs du progrès du traitement puissent être déterminés fréquemment, de façon exacte, et sans pour autant que le patient doive être durablement soumis à des conditions de traitement différentes des conditions prescrites.

Pour atteindre ce but, on prévoit, selon l'invention, un procédé de détermination d'un paramètre (Cb, D, K, Kt/V) significatif du progrès d'un traitement extracorporel de sang effectué dans un appareil de traitement de sang muni de moyens pour faire circuler le sang d'un patient et un liquide de traitement de part et d'autre de la membrane semi-perméable d'un échangeur à membrane, le procédé comprenant les étapes de :

- faire circuler successivement dans l'échangeur au moins un premier (d1) et un second (d2) liquides de traitement ayant une caractéristique (Cd) liée à au moins un des paramètres significatifs du traitement (Cb, D, K, Kt/V), la valeur de la caractéristique dans le premier liquide (d1) en amont de l'échangeur étant différente de la valeur de la caractéristique (Cd) dans le second liquide (d2) en amont de l'échangeur,
- mesurer dans chacun des premier (d1) et second (d2) liquides de traitement deux valeurs (Cd1in, Cd1out ; Cd2in, Cd2out) de la caractéristique (Cd), respectivement en amont et en aval de l'échangeur, le procédé étant caractérisé en ce qu'il comprend en outre les étapes de :
- mettre en circulation un troisième (d3) liquide de traitement dans l'échangeur alors que la caractéristique (Cd) dans le second liquide (d2) n'a pas atteint une valeur stable en aval de l'échangeur, la valeur de la caractéristique (Cd)

dans le troisième liquide (d3) en amont de l'échangeur étant différente de la valeur de la caractéristique (Cd) dans le second liquide (d2) en amont de l'échangeur,

- mesurer deux valeurs (Cd3in, Cd3out) de la caractéristique (Cd) dans le troisième liquide (d3) respectivement en amont et en aval de l'échangeur, et

- calculer une valeur d'un paramètre significatif du progrès du traitement (Cb, D, K, Kt/V) à partir des valeurs mesurées de la caractéristique (Cd) dans le premier (d1) le second (d2) et le troisième (d3) liquides de traitement.

A l'étape de calcul, au lieu de la valeur mesurée (Cd1in, Cd2in, Cd3in) de la caractéristique (Cd) dans le premier (d1), le second (d2) et le troisième (d3) liquides de traitement, il est possible d'utiliser des valeurs de consigne correspondantes (Cd1in$_{REF}$, Cd2in$_{REF}$, Cd3in$_{REF}$) qui sont fournies avant le début de chaque séance de traitement à une unité de commande pilotant la préparation du liquide de traitement.

Ce procédé présente l'intérêt de permettre une détermination précise des paramètres significatifs du progrès du traitement à partir de mesures effectuées à intervalles de temps rapprochés. De la sorte, le patient n'est exposé que pendant un temps très court à un liquide de traitement différent du liquide de traitement prescrit (par exemple trop riche ou trop pauvre en sodium) et le procédé peut être mis en oeuvre aussi souvent que nécessaire pour un suivi approprié de la séance de traitement.

Selon une caractéristique de l'invention, l'intervalle de temps (t2-ta) entre l'instant (ta), où le second liquide (d2) est mis en circulation dans l'échangeur, et l'instant (t2), où la valeur (Cd2out) de la caractéristique (Cd) dans le second liquide est mesurée en aval de l'échangeur, est choisi tel que la caractéristique (Cd) n'a pas atteint une valeur stable à l'instant (t2) en aval de l'échangeur et l'intervalle de temps (t3-tb) entre l'instant (tb), où le troisième liquide (d3) est mis en circulation dans l'échangeur, et l'instant (t3), où la valeur (Cd3out) de la caractéristique (Cd) dans le troisième liquide est mesurée en aval de l'échangeur, est choisi tel que la caractéristique (Cd) n'a pas atteint une valeur stable à l'instant (t3) en aval de l'échangeur.

Dans un mode de réalisation de l'invention, les intervalles de temps (t2-ta) et (t3-tb) sont choisis sensiblement égaux et la valeur de la caractéristique (Cd) dans le troisième liquide (d3) est choisie sensiblement égale à la valeur de la caractéristique (Cd) dans le premier liquide (d1). Le liquide de traitement prescrit peut être utilisé comme premier liquide au sens de l'invention.

Selon une autre caractéristique de l'invention, le procédé comprend en outre l'étape de calculer au moins une deuxième valeur, approchée, d'un même paramètre significatif du traitement (Cb, D, K, Kt/V). Il est alors possible de comparer ces valeurs et d'émettre un signal d'erreur si elles ne vérifient pas une loi prédéfinie. Ceci permet de contrôler qu'aucun événement indésirable n'est venu perturber les conditions de la mesure. Comme exemple de perturbation, on peut mentionner l'augmentation du débit de recirculation du sang dans le système de traitement, qui peut résulter d'un mouvement du patient, ou encore l'injection d'un liquide dans le circuit extracorporel de sang connectant le patient à l'échangeur.

D'autres caractéristiques et avantages de l'invention apparaîtront à la lecture de la description qui suit. On se reportera aux dessins sur lesquels :

La figure 1 est une représentation schématique partielle d'une installation d'hémodialyse/hémodiafiltration adaptée à la mise en oeuvre du procédé selon l'invention ; et

La figure 2 est un diagramme représentant l'évolution de la conductivité du liquide de traitement en fonction du temps lors de la mise en oeuvre du procédé selon l'invention.

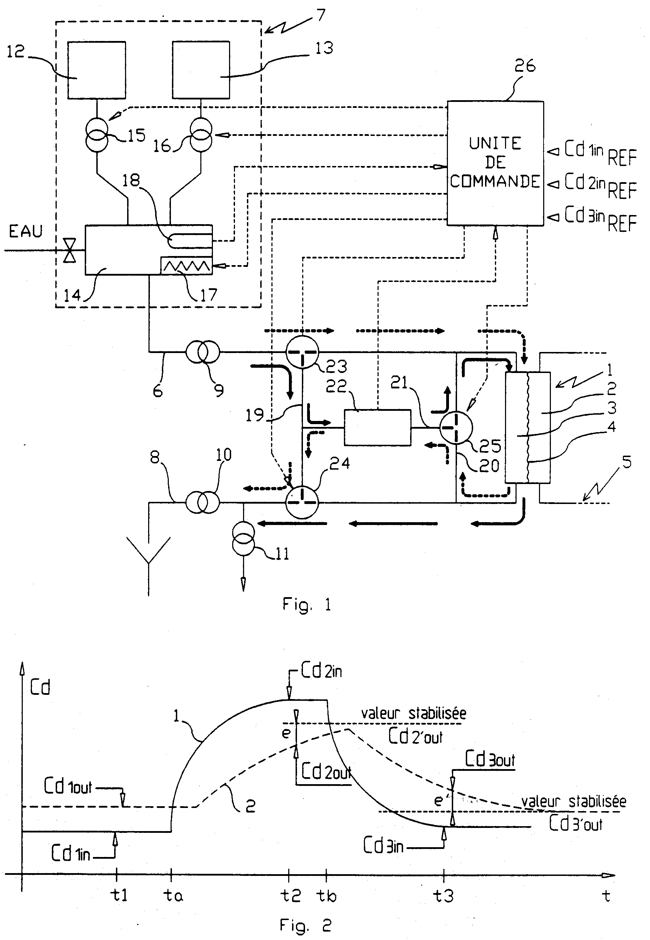

L'installation d'hémodialyse/hémodiafiltration représentée sur la figure 1 comprend un échangeur 1, tel qu'un hémodialyseur ou un hémofiltre, ayant un premier et un second compartiments 2, 3 séparés par une membrane semi-perméable 4.

Le premier compartiment 2 est connecté à un circuit 5 pour circulation extracorporelle de sang et le second compartiment 3 est connecté à un circuit de liquide de dialyse comprenant une canalisation d'alimentation 6 reliant un générateur 7 de liquide de dialyse à une entrée du second compartiment 3 et une canalisation d'évacuation 8 reliant une sortie du second compartiment 3 à un circuit d'eaux usées.

Une première pompe de circulation 9 est disposée sur la canalisation d'alimentation 6 et une seconde pompe 10 est disposée sur la canalisation d'évacuation 8, le débit de cette seconde pompe étant réglé par des moyens non représentés pour être égal au débit de la première pompe. Une pompe d'extraction 11 est connectée à la canalisation d'évacuation 8, en amont de la deuxième pompe 10, pour extraire du circuit de dialyse, le cas échéant, une quantité dosée de liquide usé correspondant à la quantité d'eau plasmatique qui est prélevée au patient par ultrafiltration.

Le générateur 7 de liquide de dialyse comprend deux réservoirs 12, 13 pour solutions concentrées reliés à un réservoir de préparation 14 destiné au mélange dosé de deux solutions concentrées avec de l'eau. Le débit d'écoulement des solutions dans le réservoir de préparation 14 peut être réglé respectivement par deux pompes 15, 16. Les solutions concentrées, qui sont de compositions complémentaires, comprennent en combinaison les principaux électrolytes du sang (sodium, potassium, calcium, magnésium, chlorures et bicarbonates). Un dispositif de chauffage 17 et

EP 0 658 352 B1

une sonde de conductivité 18 sont disposés dans le réservoir de préparation 14.

L'installation est munie aussi d'un circuit de mesure de caractéristiques du liquide de dialyse permettant d'effectuer, pour chaque caractéristique considérée, une mesure sur le liquide de dialyse frais et une mesure sur le liquide usé au moyen d'une même sonde. A cette fin, le circuit de mesure comprend une première et une seconde lignes secondaires 19, 20 montées en dérivation à l'échangeur 1 entre la ligne d'alimentation 6 et la ligne d'évacuation 8 du circuit de liquide de dialyse, ainsi qu'une ligne de jonction 21, reliant la première à la seconde ligne secondaire, dans laquelle est disposée une cellule de mesure 22 contenant une ou plusieurs sondes de mesure de caractéristiques du liquide de dialyse. La première ligne secondaire 19 est connectée respectivement aux lignes d'alimentation 6 et d'évacuation 8 au moyen de deux vannes trois-voies 23, 24 et la ligne de jonction 21 est connectée à la seconde ligne secondaire 20 au moyen d'une vanne trois-voies 25. Selon la disposition des vannes 23, 24, 25, c'est le liquide de dialyse frais (flèches en trait continu) ou le liquide usé (flèches en trait interrompu) qui circule dans la cellule de mesure 22.

Une unité de calcul et de commande 26 pilote le fonctionnement de l'installation en fonction de valeurs de consigne qui lui sont fournies initialement pour les paramètres du traitement, en particulier, le débit de sang, le débit du liquide de dialyse, le débit d'ultrafiltration, la température et la concentration électrolytique du liquide de dialyse, la durée de la séance de traitement. Dans le cadre spécifique de l'invention, l'unité de commande 26 déclenche, à intervalles réguliers, selon une séquence prédéterminée, la prise d'une série de mesures sur le liquide de dialyse. Conformément au procédé décrit plus loin, cette phase de mesure nécessite la production successive de trois liquides de dialyse de conductivités nominales différentes par le générateur de dialyse 7, la bascule des vannes 23, 24, 25 d'une position dans l'autre pour que la cellule de mesure 22 soit irriguée, pour chacun des trois liquides de dialyse, par du liquide frais et du liquide usé, et enfin le séquencement précis des prises de mesure proprement dites.

Dans un but de clarté, on a omis de représenter sur la figure 1 divers composants et accessoires d'une installation d'hémodialyse/hémodiafiltration dont la description n'aiderait pas à l'intelligence de l'invention, tels que, notamment, des moyens de mesure de pression dans les différents circuits et des moyens de mesure de débit Qd du liquide de dialyse et et de mesure de débit Quf d'ultrafiltration.

Comme cela a été rappelé plus haut, le principe de l'invention consiste à mesurer certaines caractéristiques du liquide de traitement (liquide de dialyse) afin d'en déduire par le calcul la valeur réelle de caractéristiques correspondantes du sang, ainsi que la valeur réelle de paramètres significatifs de l'efficacité du traitement, liés à ces caractéristiques du sang.

La cellule de mesure 22 peut ainsi contenir une sonde de température, une sonde de conductivité, une électrode de mesure de concentration en tel ou tel soluté, une sonde de pH et de mesure de pression partielle de $CO_2$, etc.

Dans la suite on décrira le procédé selon l'invention en prenant l'exemple, qui ne saurait être considéré comme limitatif, d'une mesure de conductivité. On rappelle qu'il existe une excellente corrélation entre la conductivité du liquide de dialyse et sa concentration en substances ionisées, dont le sodium représente la part prépondérante. C'est grâce à cette corrélation qu'il est possible de calculer la concentration réelle (Cbin) en sodium du sang à l'entrée de l'échangeur à partir de quatre valeurs mesurées de la conductivité du liquide de dialyse (Cd1in, Cd2in, Cd1out, Cd2out, respectivement conductivité à l'entrée et à la sortie de l'échangeur, mesurée lors du passage successif d'un premier et d'un second liquide de dialyse d1, d2 ayant des conductivités différentes), par application de la formule :

$$Cbin = \frac{Cd2out \times Cd1in - Cd1out \times Cd2in}{(Cd1in - Cd1out) - (Cd2in - Cd2out)} \tag{1}$$

laquelle est déduite de la formule générale de la dialysance D :

$$D = \frac{-(Qd + Quf) \times (Cdin - Cdout)}{Cbin - Cdin} \tag{2}$$

où Qd est le débit de liquide dans le compartiment de l'échangeur connecté au circuit de dialyse, Quf est le débit d'ultrafiltration, Cdin et Cdout la conductivité/concentration en substances ionisées du liquide de dialyse en amont et en aval de l'échangeur et Cbin, la concentration en sodium du sang en amont de l'échangeur.

Connaissant la valeur réelle de la concentration Cbin du sodium dans le sang à l'entrée de l'échangeur, on peut calculer la dialysance réelle D du système au moyen de la formule (2).

Sachant par ailleurs qu'il existe des tables de correspondance entre la dialysance pour le sodium et la clairance pour l'urée, on peut déduire la clairance réelle K de l'échangeur pour l'urée de la dialysance réelle calculée D.

Enfin, à partir de la clairance réelle K, de la durée écoulée du traitement t et du volume V de répartition de l'urée dans le patient (qui dépend du poids moyen, du sexe et de l'âge), on peut calculer la dose de dialyse administrée Kt/V.

Conformément à l'invention, afin d'éviter de soumettre le patient à des conditions de traitement hors de la normale (liquide de dialyse ayant une conductivité supérieure ou inférieure à la conductivité prescrite) pendant un temps significatif, on effectue trois séries de mesures de conductivité rapprochées sur trois liquides de dialyse d1, d2, d3 de conductivités nominales différentes, les mesures sur le second et le troisième liquides de dialyse d2, d3 en aval de l'échangeur étant prises avant que cette conductivité ne se soit stabilisée. La façon dont les différents paramètres significatifs de l'évolution du traitement peuvent être calculés exactement à partir de mesures prises pendant un état transitoire du liquide de dialyse pour ce qui est de la conductivité va être expliquée maintenant en relation avec la figure 2.

La figure 2 représente deux graphes de la conductivité du liquide de dialyse en fonction du temps, le graphe 1 en trait continu correspond à l'évolution de la conductivité en amont de l'échangeur et le graphe 2 en trait interrompu correspond à l'évolution de la conductivité en aval de l'échangeur.

Conformément à ces graphes, un premier liquide de dialyse d1 de conductivité constante préparé par le générateur 7 est mis en circulation dans l'échangeur 1 jusqu'à un instant ta. La conductivité du liquide de dialyse d1, en amont de l'échangeur 1, est, en principe, sensiblement égale à la valeur de consigne correspondante fournie à l'unité de commande 26 avant le début de la séance de traitement.

Le liquide de dialyse d1 est de préférence celui dont la composition électrolytique a été prescrite initialement par le médecin. On remarque que la conductivité en aval du dialyseur est supérieure à la conductivité en amont, ce qui signifie qu'un transfert de substances ionisées se produit dans l'échangeur, du sang vers le liquide de dialyse. Antérieurement à l'instant ta, on mesure successivement, grâce au dispositif de mesure représenté sur la figure 1, la conductivité $Cd1in$ du premier liquide de dialyse en amont de l'échangeur 1, puis la conductivité $Cd1out$ en aval de l'échangeur, cette seconde mesure étant prise à l'instant t1. Les valeurs mesurées $Cd1in$ et $Cd1out$ sont mises en mémoire dans une mémoire de l'unité de commande et de calcul 26.

Le générateur 7 de liquide de dialyse est programmé ensuite pour produire, pendant une courte période de temps tb - ta (deux minutes par exemple), un second liquide de dialyse d2 différant du premier liquide de dialyse d1 par une conductivité nominale plus élevée, également constante. La conductivité du liquide de dialyse d2, en amont de l'échangeur 1, est, en principe, sensiblement égale à la valeur de consigne correspondante $Cd2in_{REF}$ fournie à l'unité de commande 26 avant le début de la séance de traitement. Ce qui circule alors dans le circuit de dialyse est pour un temps un mélange du premier et du second liquides de dialyse d1, d2, où la proportion du premier diminue graduellement, ce que traduisent les graphes par une courbe ascendante s'aplatissant rapidement pour tendre vers une asymptote horizontale. On remarque que la conductivité en amont du dialyseur (graphe 1) se stabilise rapidement, avant l'instant tb, alors que la conductivité en aval de l'échangeur (graphe 2) tend plus lentement vers une valeur constante, qu'elle n'a pas atteinte à l'instant tb. Cela est dû au fait que la conductivité en aval de l'échangeur ne cesse pas d'évoluer aussi longtemps que l'échangeur n'a pas été totalement purgé du mélange résultant de la mise en circulation du second liquide de dialyse et aussi longtemps que les phénomènes de transfert diffusifs n'ont pas atteint un régime d'équilibre dans l'échangeur.

On remarque aussi, dans la portion ascendante des graphes, que la conductivité du liquide de dialyse à l'entrée de l'échangeur est supérieure à la conductivité du liquide de dialyse à la sortie de l'échangeur, ce qui traduit le fait qu'un transfert diffusif de substances ionisées a lieu cette fois du liquide de dialyse vers le sang.

Antérieurement à l'instant tb, on mesure successivement, au moyen du dispositif de mesure représenté sur la figure 1, la conductivité $Cd2in$ du second liquide de dialyse en amont de l'échangeur 1, puis la conductivité $Cd2out$ en aval de l'échangeur, cette seconde mesure étant prise à l'instant t2. Il existe une différence e entre la valeur de la conductivité $Cd2out$ mesurée en aval de l'échangeur 1 et ce qui serait la valeur exacte ou stabilisée $Cd2'out$ de la conductivité du second liquide de dialyse d2 en aval de l'échangeur si le second liquide de dialyse d2 était mis en circulation suffisamment longtemps dans le circuit de liquide de dialyse, ce qui, pour les raisons mentionnées plus haut, est indésirable. Les valeurs mesurées $Cd2in$ et $Cd2out$ sont mises en mémoire dans une mémoire de l'unité de commande et de calcul 26.

A partir de l'instant tb, le générateur de liquide de dialyse 7 est programmé pour produire un troisième liquide de dialyse d3 ayant une conductivité nominale constante inférieure à la conductivité du second liquide de dialyse d2. La conductivité du liquide de dialyse d3, en amont de l'échangeur 1, est en principe, sensiblement égale à la valeur de consigne correspondante $Cd3in_{REF}$ fournie à l'unité de commande 26 avant le début de la séance de traitement. Il circule alors pour un temps dans le circuit de dialyse un mélange du second et du troisième liquide de dialyse d2, d3, où la proportion du troisième liquide de dialyse d3 augmente rapidement, ce que traduisent les graphes par une courbe descendante s'aplatissant pour tendre vers une asymptote horizontale correspondant à la valeur de la conductivité du troisième liquide de dialyse d3 respectivement en amont (graphe 1) et en aval (graphe 2) de l'échangeur. Pour les mêmes raisons que lors de la phase de circulation du deuxième liquide de dialyse, la conductivité en amont de l'échangeur se stabilise plus vite que la conductivité en aval de l'échangeur pour finir par atteindre la valeur de la conductivité du troisième liquide de dialyse d3. On remarque que, peu après l'instant tb, il se produit un renversement du sens du transfert diffusif des substances ionisées dans l'échangeur, ces substances migrant à nouveau du sang vers le liquide de dialyse.

Lorsque la conductivité en amont de l'échangeur est stabilisée et alors que la condvctivité en aval de l'échangeur décroît toujours. on mesure successivement la valeur de ces conductivités, respectivement $Cd3in$ et $Cd3out$, la mesure de la conductivité en aval de l'échangeur étant prise à l'instant t3 et cette valeur différant de la valeur stabilisée $Cd3'out$

(conductivité du troisième liquide de dialyse d3) d'une quantité e'. Les valeurs mesurées Cd3in et Cd3out sont sont mises en mémoire dans une mémoire de l'unité de commande et de calcul 26.

A l'issue de l'étape de prise de mesures qui vient d'être décrite, l'unité de commande et de calcul a donc en mémoire six valeurs mesurées Cd1in, Cd1out, Cd2in, Cd2out, Cd3in, Cd3out, qui ne sont pas directement exploitables par application de la formule 1, laquelle n'est exacte que pour les valeurs de conductivité stabilisées.

Selon l'invention, pour calculer la concentration Cbin du sang en sodium en amont de l'échangeur ou la dialysance réelle D du système, on part de l'observation que les quantités e, e' ne dépendent que de trois facteurs : l'amplitude de la perturbation (Cd2in-Cd1in, Cd3in-Cd2in) induite dans le système par la mise en circulation du second liquide de dialyse d2 et du troisième liquide de dialyse d3 ; l'instant de la mesure (t2, t3) par rapport au début de la perturbation (ta, tb) ; et la constante de temps θ du système, qui dépend des débits du liquide de dialyse et du sang, de la surface de la membrane 4 et du coefficient de diffusion de la membrane pour le soluté considéré, ici le sodium.

C'est ainsi qu'en choisissant deux perturbations dont les amplitudes soient égales en valeur absolue, ainsi que des intervalles de temps entre le début de chaque perturbation et la mesure correspondante qui soient égaux ( t2 - ta = t3 - tb ), on est assuré que les quantités e et e' sont égales, de sorte que, par application de la formule (2), le calcul de Cbin ou de D se fera simplement par la résolution de trois équations à trois inconnues, Cbin, D et e, les débits de liquide de dialyse et d'ultrafiltration Qd et Quf étant connus par ailleurs. D'autres paramètres significatifs du progrès de la séance de dialyse, tels que la clairance K ou la dose de dialyse Kt/V seront déduits immédiatement du calcul de la concentration réelle du sodium dans le sang Cbin ou de la dialysance réelle D.

A titre d'example, le système d'équations mentionné plus haut peut être mis sous la forme suivante :

$$Cbin = \frac{Cd1out - (1 - A) \times Cd1in}{A}$$

$$D = (Qd + Quf) \times A$$

où

$$A = 1 - \frac{(2 \times Cd1out - Cd2out - Cd3out)}{2 \times Cd1in - Cd2in - Cd3in}$$

Par ailleurs, selon l'invention, on calcule plusieurs valeurs du même paramètre de façon à vérifier si une perturbation accidentelle est intervenue pendant l'étape de prise de mesures décrite plus haut. Par exemple, on peut calculer trois valeurs de la dialysance D, une première valeur exacte D0 en fonction des six mesures de conductivité, une second valeur approchée D1 en fonction de Cd1in, Cd1out, Cd2in, Cd2out et une troisième valeur approchée D2 en fonction de Cd2in, Cd2out, Cd3in, Cd3out. Ensuite, on contrôle si les trois valeurs ainsi calculées obéissent à une ou plusieurs lois prédéfinies telles que : D0 inférieur ou égal à D1 et D1 inférieur ou égal à D2, ou encore la différence D0-D1 est égale ou sensiblement égale à la différence D1-D2. Dans le cas où une de ces lois ne serait pas respectée, un signal d'erreur est émis.

L'invention qui vient d'être décrite est susceptible de variantes, à la fois en ce qui concerne l'étape de prise de mesures proprement dite (choix de la valeur de la conductivité nominale des différents liquides de dialyse, c'est-à-dire aussi amplitude et sens de la perturbation induite dans le système, et choix de l'instant où les mesures sont prises en fonction du début des perturbations successives) et façon dont les paramètres significatifs du déroulement du traitement sont calculés à partir des valeurs de conductivité mesurées.

En ce qui concerne le choix du sens de l'échelon de conductivité entre deux liquides de dialyse mis en circulation successivement, toutes les combinaisons sont possibles. A titre d'exemple :

La valeur de la caractéristique Cd dans le premier liquide d1 est choisie inférieure à la valeur de la caractéristique Cd dans le second liquide d2, laquelle est elle-même choisie inférieure à la valeur de la caractéristique Cd dans le troisième liquide d3.

La valeur de la caractéristique Cd dans le premier liquide d1 est choisie supérieure à la valeur de la caractéristique Cd dans le second liquide d2, laquelle est elle-même choisie supérieure à la valeur de la caractéristique Cd dans le troisième liquide d3.

La valeur de la caractéristique Cd dans le premier liquide d1 est choisie inférieure à la valeur de la caractéristique Cd dans le second liquide d2, laquelle est elle-même choisie supérieure à la valeur de la caractéristique Cd dans le troisième liquide d3.

La valeur de la caractéristique Cd dans le premier liquide d1 est choisie supérieure à la valeur de la caractéristique Cd dans le second liquide d2, laquelle est elle-même choisie inférieure à la valeur de la caractéristique Cd dans le troisième liquide d3.

La valeur de la caractéristique Cd dans le second liquide d2 en amont de l'échangeur est choisie inférieure ou supérieure à la valeur de la caractéristique Cd dans le premier liquide d1 en amont de l'échangeur, en fonction du signe de la différence (Cdin1-Cdout1) entre les valeurs mesurées de la caractéristique Cd dans le premier liquide d1 en amont et en aval de l'échangeur.

La valeur de la caractéristique Cd dans le troisième liquide d3 en amont de l'échangeur est choisie inférieure ou supérieure à la valeur de la caractéristique Cd dans le second liquide d2 en amont de l'échangeur, en fonction du signe de la différence (Cdin1-Cdout1) entre les valeurs mesurées de de la caractéristique Cd dans le premier liquide d1 en amont et en aval de l'échangeur et/ou en fonction du signe de la différence (Cdin2-Cdout2) entre les valeurs mesurées de de la caractéristique Cd dans le second liquide d2 en amont et en aval de l'échangeur.

La valeur de la caractéristique Cd dans le troisième liquide d3 en amont de l'échangeur est choisie en fonction d'une valeur approchée de la concentration en sodium dans le sang Cb', calculée en fonction des valeurs mesurées Cd1in, Cd1out, Cd2in, Cd2out sur les premier et second liquides de dialyse d1, d2 : par exemple, la valeur de la caractéristique Cd est choisie égale ou très différente de Cb'.

Dans tous les exemples précités, le premier liquide de dialyse peut être le liquide de dialyse prescrit pour le traitement. A l'exception des deux premiers exemples, le troisième liquide de dialyse peut être choisi sensiblement identique au liquide de dialyse prescrit pour le traitement.

En ce qui concerne l'amplitude des échelons de conductivité (Cd2in-Cd1in, Cd3in-Cd2in) et la durée des intervalles de temps entre le début de chaque perturbations et l'instant de la mesure correspondante (t2-ta, t3-tb), toutes les combinaisons sont encore possibles, pourvu que ces échelons et ces intervalles soient suffisamment importants pour que les valeurs mesurées soient significatives.

A titre d'exemple, les deux échelons de conductivité et/ou les deux intervalles de mesures peuvent être choisis pour que, bien que respectivement d'amplitudes et/ou de durées différentes, les quantités e et e' soient sensiblement égales, auquel cas la méthode de calcul exposée plus haut est applicable.

S'il résultait au contraire que, par suite de ces choix, les quantités e et e' ne soient pas égales, une méthode de calcul pourrait consister à mesurer la valeur stabilisée Cd3'out du troisième liquide de dialyse d3 en aval de l'échangeur, à calculer

$$e' = Cd3out - Cd3'out,$$

puis à calculer e en fonction de e', grâce à quoi la valeur stabilisée du deuxième liquide de dialyse d2 en aval de l'échangeur, inaccessible à la mesure, pourrait être calculée. On disposerait alors de trois couples de valeurs mesurées/calculées de la conductivité des trois liquides de dialyse utilisables deux à deux dans l'équation (1). Une autre méthode de calcul consiste à résoudre le système d'équations suivant :

$$Cbin = \frac{Cd1out - (1 - A) \times Cd1in}{A}$$

$$D = (Qd + Quf) \times A$$

où

$$A = 1 - \frac{(2 \times Cd1out - Cd2'out - Cd3'out)}{(2 \times Cd1in - Cd2in - Cd3in)}$$

$$Cd1out = (1 - A) Cd1in + ACbin$$

$$Cd2'out = Cd2out + e$$
$$= Cd1out + (1 - A)(Cd2in - Cd1in)(1 - \exp(ta - t2)/\theta)$$

$$Cd3'out = Cd3out - e'$$
$$= Cd1out + (1 - A)(Cd2in - Cd1in)(1 - \exp(ta - t3)/\theta) + (1 - A)(Cd3in - Cd2in)(1 - \exp(tb - t3)/\theta)$$

$\theta$ étant la constante de temps du système, qui, comme cela a été mentionné plus haut, dépend des débits du liquide de dialyse et du sang. de la surface de la membrane 4 et du coefficient de diffusion de la membrane pour le soluté considéré.

L'invention n'est pas limitée aux exemples de réalisation qui viennent d'être décrits et elle est susceptible de variantes. Par exemple, dans les étapes de calcul du procédé, qui font intervenir la valeur de la conductivité des liquides de dialyse d1, d2, d3 en amont du dialyseur, il est possible d'utiliser, au lieu de valeurs mesurées Cd1in, Cd2in, Cd3in, les valeurs de consigne correspondantes $Cd1in_{REF}$, $Cd2in_{REF}$, $Cd3in_{REF}$ qui sont fournies à l'unité de commande 26 avant le début de chaque séance de traitement.

On comprendra aussi que le procédé selon l'invention peut être mis en oeuvre sur d'autres installations que l'installation décrite ci-dessus. Le générateur de liquide de dialyse pourrait être un générateur en ligne. De même, au lieu d'utiliser une même sonde pour prendre des mesures à la fois sur le liquide de dialyse frais et sur le liquide usé, on pourrait disposer une sonde en amont de l'échangeur et un sonde en aval.

## Revendications

1. Procédé de détermination d'un paramètre (Cb, D, K, Kt/V) significatif du progrès d'un traitement extracorporel de sang effectué dans un appareil de traitement de sang muni de moyens pour faire circuler le sang d'un patient et un liquide de traitement de part et d'autre de la membrane (4) semi-perméable d'un échangeur à membrane (1), le procédé comprenant les étapes de :

   - faire circuler successivement dans l'échangeur au moins un premier (d1) et un second (d2) liquides de traitement ayant une caractéristique Cd liée à au moins un des paramètres significatifs du traitement (Cb, D, K, Kt/V), la valeur de la caractéristique dans le premier liquide (d1) en amont de l'échangeur étant différente de la valeur de la caractéristique Cd dans le second liquide (d2) en amont de l'échangeur,
   - mesurer dans chacun des premier (d1) et second (d2) liquides de traitement deux valeurs Cd1in, Cd1out ; Cd2in, Cd2out de la caractéristique Cd, respectivement en amont et en aval de l'échangeur ;

   le procédé étant caractérisé en ce qu'il comprend en outre les étapes de :

   - mettre en circulation un troisième (d3) liquide de traitement dans l'échangeur alors que la caractéristique Cd du second liquide (d2) n'a pas atteint une valeur stable en aval de l'échangeur, la valeur de la caractéristique Cd dans le troisième liquide (d3) en amont de l'échangeur étant différente de la valeur de la caractéristique Cd dans le second liquide (d2) en amont de l'échangeur,
   - mesurer deux valeurs Cd3in, Cd3out de la caractéristique Cd dans le troisième liquide (d3) respectivement en amont et en aval de l'échangeur, et
   - calculer au moins une valeur d'au moins un paramètre significatif du progrès du traitement (Cb, D, K, Kt/V) à partir des valeurs mesurées de la caractéristique Cd dans le premier (d1) le second (d2) et le troisième (d3) liquides de traitement.

2. Procédé selon la revendication 1, caractérisé en ce que

   - l'intervalle de temps t2-ta entre l'instant ta, où le second liquide (d2) est mis en circulation dans l'échangeur, et l'instant t2, où la valeur Cd2out de la caractéristique Cd dans le second liquide est mesurée en aval de l'échangeur, est choisi tel que la caractéristique Cd n'a pas atteint une valeur stable à l'instant t2 en aval de l'échangeur et
   - l'intervalle de temps t3-tb entre l'instant tb, où le troisième liquide (d3) est mis en circulation dans l'échangeur, et l'instant t3, où la valeur Cd3out de la caractéristique Cd dans le troisième liquide est mesurée en aval de l'échangeur, est choisi tel que la caractéristique Cd n'a pas atteint une valeur stable à l'instant t3 en aval de l'échangeur.

3. Procédé selon une des revendications 1 et 2, caractérisé en ce que, en amont de l'échangeur, la valeur de la caractéristique Cd dans le premier liquide (d1) est choisie inférieure à la valeur de la caractéristique Cd dans le second liquide (d2), laquelle est elle-même choisie inférieure à la valeur de la caractéristique Cd dans le troisième liquide (d3).

4. Procédé selon une des revendications 1 et 2, caractérisé en ce que, en amont de l'échangeur, la valeur de la caractéristique Cd dans le premier liquide (d1) est choisie supérieure à la valeur de la caractéristique Cd dans le second liquide (d2), laquelle est elle-même choisie supérieure à la valeur de la caractéristique Cd dans le troisième liquide (d3).

5. Procédé selon une des revendications 1 et 2, caractérisé en ce que, en amont de l'échangeur, la valeur de la caractéristique Cd dans le premier liquide (d1) est choisie inférieure à la valeur de la caractéristique Cd dans le second liquide (d2), laquelle est elle-même choisie supérieure à la valeur de la caractéristique Cd dans le troisième liquide (d3).

6. Procédé selon une des revendications 1 et 2, caractérisé en ce que, en amont de l'échangeur, la valeur de la caractéristique Cd dans le premier liquide (d1) est choisie supérieure à la valeur de la caractéristique Cd dans le second liquide (d2), laquelle est elle-même choisie inférieure à la valeur de la caractéristique Cd dans le troisième liquide (d3).

7. Procédé selon une des revendications 1 et 2, caractérisé en ce que la valeur de la caractéristique Cd dans le second liquide (d2) en amont de l'échangeur est choisie inférieure ou supérieure à la valeur de la caractéristique Cd dans le premier liquide (d1) en amont de l'échangeur, en fonction du signe de la différence Cdin1-Cdout1 entre les valeurs mesurées de la caractéristique Cd dans le premier liquide (d1) en amont et en aval de l'échangeur.

8. Procédé selon une des revendications 1 et 2, caractérisé en ce que la valeur de la caractéristique Cd dans le troisième liquide (d3) en amont de l'échangeur est choisie inférieure ou supérieure à la valeur de la caractéristique Cd dans le second liquide (d2) en amont de l'échangeur, en fonction du signe de la différence Cdin1-Cdout1 entre les valeurs mesurées de la caractéristique Cd dans le premier liquide (d1) en amont et en aval de l'échangeur et/ou en fonction du signe de la différence Cdin2-Cdout2 entre les valeurs mesurées de la caractéristique Cd dans le second liquide (d2) en amont et en aval de l'échangeur.

9. Procédé selon une des revendications 1 et 2, caractérisé en ce que la valeur de la caractéristique Cd dans le troisième liquide (d3) en amont de l'échangeur est choisie en fonction d'une valeur approchée de la concentration en sodium dans le sang Cb', calculée en fonction des valeurs mesurées Cd1in, Cd1out, Cd2in, Cd2out sur les premier et second liquides de dialyse (d1, d2).

10. Procédé selon une des revendications 5 à 8, caractérisé en ce que, en amont de l'échangeur, la valeur de la caractéristique Cd dans le troisième liquide (d3) est choisie sensiblement égale à la valeur de la caractéristique Cd dans le premier liquide (d1).

11. Procédé selon une des revendications 2 à 10, caractérisé en ce que les intervalles de temps t2-ta et t3-tb sont choisis sensiblement égaux.

12. Procédé selon une des revendications 1 à 11, caractérisé en ce qu'il comprend en outre l'étape de calculer au moins une deuxième valeur du paramètre significatif du traitement (Cb, D, K, Kt/V).

13. Procédé selon la revendication 12, caractérisé en ce qu'il comprend en outre les étapes de :

- comparer au moins deux valeurs du paramètre significatif du traitement (Cb, D, K, Kt/V) ; et
- émettre un signal d'erreur si les valeurs du paramètre significatif du traitement (Cb, D, K, Kt/V) ne vérifient pas une loi prédéfinie.

14. Procédé selon une des revendications 1 à 13, caractérisé en ce que la caractéristique du liquide de traitement (d) qui est mesurée est la conductivité ou la concentration en sodium Cd et le paramètre significatif du traitement qui est calculé est la concentration du sodium Cb dans le sang en amont de l'échangeur.

15. Procédé selon les revendications 10, 11 et 14 caractérisé en ce que la concentration du sodium Cb dans le sang en amont de l'échangeur est calculée selon la formule :

$$Cb = \frac{Cd1out - (1 - A) \times Cd1in}{A}$$

où

$$A = 1 - \frac{(2 \times Cd1out - Cd2out - Cd3out)}{(2 \times Cd1in - Cd2in - Cd3in)}$$

16. Procédé selon une des revendications 1 à 13, caractérisé en ce que la caractéristique du liquide de traitement (d) qui est mesurée est la conductivité ou la concentration en sodium Cd et le paramètre significatif du traitement qui est calculé est la dialysance D.

17. Procédé selon les revendications 10, 11 et 16 caractérisé en ce que la dialysance D est calculée selon la formule :

$$D = (Qd + Quf) \times A$$

où

Qd est le débit du liquide de dialyse,
Quf est le débit d'ultrafiltration, et

$$A = 1 - \frac{(2 \times Cd1out - Cd2out - Cd3out)}{(2 \times Cd1in - Cd2in - Cd3in)}$$

**18.** Procédé selon une des revendications 16 ou 17, caractérisé en ce que la clairance K pour un métabolite du sang (urée, créatinine, etc.) est déduite de la dialysance D à partir d'une table de correspondance préalablement établie.

**19.** Procédé selon la revendication 18, caractérisé en ce que le paramètre significatif du traitement qui est calculé est la dose de dialyse Kt/V, où t est le temps de traitement écoulé et V est le volume d'eau total du patient.

**20.** Procédé selon une des revendications 1 à 19, caractérisé en ce que, à l'étape de calculer au moins une valeur d'au moins un paramètre significatif du progrès du traitement (Cb, D, K, Kt/V), au lieu de la valeur mesurée Cd1in, Cd2in, Cd3in de la caractéristique Cd dans le premier (d1), le second (d2) et le troisième (d3) liquides de traitement, on utilise des valeurs de consigne correspondantes $Cd1in_{REF}$, $Cd2in_{REF}$, $Cd3in_{REF}$ qui sont fournies avant le début de chaque séance de traitement à une unité de commande (26) pilotant la préparation du liquide de traitement.

## Claims

**1.** Method for the determination of a significant parameter (Cb, D, K, Kt/V) of the progress of an extracorporeal treatment of blood carried out in an apparatus for treating blood provided with means for circulating the blood of a patient and a treatment fluid on both sides of the semipermeable membrane (4) of a membrane exchanger (1),

the method comprising steps of:

- successively circulating in the exchanger at least a first (d1) and a second (d2) treatment fluid having a characteristic Cd linked to at least one of the significant parameters of the treatment (Cb, D, K, Kt/V), the value of the characteristic in the first fluid (d1) upstream of the exchanger being different from the value of the characteristic Cd in the second fluid (d2) upstream of the exchanger,
- measuring in each of the first (d1) and second (d2) treatment fluids two values (Cd1in, Cd1out; Cd2in, Cd2out) of the characteristic Cd, respectively upstream and downstream of the exchanger,

the method being characterized in that it comprises, in addition, the steps of:

- putting into circulation a third (d3) treatment fluid in the exchanger while the characteristic Cd of the second fluid (d2) has not reached a stable value downstream of the exchanger, the value of the characteristic Cd in the third fluid (d3) upstream of the exchanger being different from the value of the characteristic Cd in the second fluid (d2) upstream of the exchanger,
- measuring two values (Cd3in, Cd3out) of the characteristic Cd in the third fluid (d3) respectively upstream and downstream of the exchanger, and
- calculating at least a value of at least a significant parameter of the progress of the treatment (Cb, D, K, Kt/V) from the measured values of the characteristic Cd in the first (d1), the second (d2) and the third (d3) treatment fluids.

**2.** Method according to Claim 1, characterized in that

- the time interval t2-ta between the instant ta, where the second fluid (d2) is put into circulation in the exchanger, and the instant t2, where the value Cd2out of the characteristic Cd in the second fluid is measured downstream of the exchanger, is chosen such that the characteristic Cd has not reached a stable value at the instant t2 downstream of the exchanger and
- the time interval t3-tb between the instant tb, where the third fluid (d3) is put into circulation in the exchanger, and the instant t3, where the value Cd3out of the characteristic Cd in the third fluid is measured downstream of the exchanger, is chosen such that the characteristic Cd has not reached a stable value at the instant t3 downstream of the exchanger.

3. Method according to either of Claims 1 and 2, characterized in that, upstream of the exchanger, the value of the characteristic Cd in the first fluid (d1) is chosen less than the value of the characteristic Cd in the second fluid (d2), which is itself chosen less than the value of the characteristic Cd in the third fluid (d3).

4. Method according to either of Claims 1 and 2, characterized in that, upstream of the exchanger, the value of the characteristic Cd in the first fluid (d1) is chosen greater than the value of the characteristic Cd in the second fluid (d2), which is itself chosen greater than the value of the characteristic Cd in the third fluid (d3).

5. Method according to either of Claims 1 and 2, characterized in that, upstream of the exchanger, the value of the characteristic Cd in the first fluid (d1) is chosen less than the value of the characteristic Cd in the second fluid (d2), which is itself chosen greater than the value of the characteristic Cd in the third fluid (d3).

6. Method according to either of Claims 1 and 2, characterized in that, upstream of the exchanger, the value of the characteristic Cd in the first fluid (d1) is chosen greater than the value of the characteristic Cd in the second fluid (d2), which is itself chosen less than the value of the characteristic Cd in the third fluid (d3).

7. Method according to either of Claims 1 and 2, characterized in that the value of the characteristic Cd in the second fluid (d2) upstream of the exchanger is chosen less than or greater than the value of the characteristic Cd in the first fluid (d1) upstream of the exchanger, according to the sign of the difference Cdin1-Cdout1 between the measured values of the characteristic Cd in the first fluid (d1) upstream and downstream of the exchanger.

8. Method according to either of Claims 1 and 2, characterized in that the value of the characteristic Cd in the third fluid (d3) upstream of the exchanger is chosen less than or greater than the value of the characteristic Cd in the second fluid (d2) upstream of the exchanger, according to the sign of the difference Cdin1-Cdout1 between the measured values of the characteristic Cd in the first fluid (d1) upstream and downstream of the exchanger and/or according to the sign of the difference Cdin2-Cdout2 between the measured values of the characteristic Cd in the second fluid (d2) upstream and downstream of the exchanger.

9. Method according to either of Claims 1 and 2, characterized in that the value of the characteristic Cd in the third fluid (d3) upstream of the exchanger is chosen as a function of an approximate value of the blood sodium concentration (Cb'), calculated as a function of the measured values Cd1in, Cd1out, Cd2in, Cd2out on the first and second dialysis fluids (d1, d2).

10. Method according to one of Claims 5 to 8, characterized in that, upstream of the exchanger, the value of the characteristic Cd in the third fluid (d3) is chosen substantially equal to the value of the characteristic Cd in the first fluid (d1).

11. Method according to one of Claims 2 to 10, characterized in that the time intervals t2-ta and t3-tb are chosen substantially equal.

12. Method according to one of Claims 1 to 11, characterized in that it comprises, in addition, the step of calculating at least a second value of the significant parameter of the treatment (Cb, D, K, Kt/V).

13. Method according to Claim 12, characterized in that it comprises, in addition, the steps of:

   - comparing at least two values of the significant parameter of the treatment (Cb, D, K, Kt/V); and
   - emitting an error signal if the values of the significant parameter of the treatment (Cb, D, K, Kt/V) do not confirm a predefined law.

14. Method according to one of Claims 1 to 13, characterized in that the characteristic of the treatment fluid (d) which is measured is the conductivity or the sodium concentration Cd and the significant parameter of the treatment which is calculated is the concentration of sodium Cb in the blood upstream of the exchanger.

15. Method according to Claims 10, 11 and 14, characterized in that the concentration of sodium Cb in the blood upstream of the exchanger is calculated according to the formula:

$$Cb = \frac{Cd1out - (1 - A) \times Cd1in}{A}$$

where

$$A = 1 - \frac{(2 \times Cd1out - Cd2out - Cd3out)}{(2 \times Cd1in - Cd2in - Cd3in)}$$

**16.** Method according to one of Claims 1 to 13, characterized in that the characteristic of the treatment fluid (d) which is measured is the conductivity or the sodium concentration Cd and the significant parameter of the treatment which is calculated is the dialysance D.

**17.** Method according to Claims 10, 11 and 16, characterized in that the dialysance D is calculated according to the formula:

$$D = (Qd + Quf) \times A$$

where

Qd is the flow rate of the dialysis fluid,

Quf is the rate of ultrafiltration, and

$$A = 1 - \frac{(2 \times Cd1out - Cd2out - Cd3out)}{(2 \times Cd1in - Cd2in - Cd3in)}$$

**18.** Method according to either of Claims 16 and 17, characterized in that the clearance K for a blood metabolite (urea, creatinine, and the like) is deduced from the dialysance D from a correspondence table established beforehand.

**19.** Method according to Claim 18, characterized in that the significant parameter of the treatment which is calculated is the dose of dialysis Kt/V, where t is the treatment time elapsed and V is the total volume of water for the patient.

**20.** Method according to one of Claims 1 to 20, characterized in that, at the step of calculating at least a value of at least a significant parameter of the progress of the treatment (Cb, D, K, Kt/V), instead of measured values Cd1in, Cd2in, Cd3in of the characteristic Cd in the first (d1), the second (d2) and the third (d3) treatment liquids, there are used corresponding reference values $Cd1in_{REF}$, $Cd2in_{REF}$, $Cd3in_{REF}$, which are entered before each treatment session in a control unit controlling the preparation of the treatment liquid.

**Patentansprüche**

**1.** Verfahren zur Bestimmung eines Parameters (Cb, D, K, Kt/V), der den Fortschritt einer extrakorporalen Blutbehandlung kennzeichnet, die in einer Vorrichtung zur Blutbehandlung durchgeführt wird, die mit Mitteln versehen ist, um das Blut eines Patienten und eine Behandlungsflüssigkeit auf beiden Seiten der semipermeablen Membran (4) eines Austauschers mit Membran (1) zirkulieren zu lassen,
wobei das Verfahren die folgenden Schritte aufweist:

- in dem Austauscher wenigstens eine erste (d1) und eine zweite (d2) Behandlungsflüssigkeit zirkulieren zu lassen, die eine Eigenschaft Cd aufweisen, die mit wenigstens einem der die Behandlung kennzeichnenden Parameter (Cb, D, K, Kt/V) verknüpft ist, wobei der Wert der Eigenschaft in der ersten Flüssigkeit (d1) stromauf vom Austauscher von dem Wert der Eigenschaft Cd in der zweiten Flüssigkeit (d2) stromauf vom Austauscher verschieden ist,
- das Messen zweier Werte (Cd1in, Cd1out; Cd2in, Cd2out) der Eigenschaft Cd in jeder der ersten (d1) und zweiten (d2) Behandlungsflüssigkeiten stromauf beziehungsweise stromab vom Austauscher;

wobei das Verfahren dadurch gekennzeichnet ist, daß es außerdem die folgenden Schritte aufweist:

- eine dritte Behandlungsflüssigkeit (d3) in dem Austauscher in Umlauf zu bringen, während die Eigenschaft Cd der zweiten Flüssigkeit (d2) stromab vom Austauscher noch keinen stabilen Wert angenommen hat, wobei der Wert der Eigenschaft Cd in der dritten Flüssigkeit (d3) stromauf vom Austauscher vom Wert der Eigenschaft Cd in der zweiten Flüssigkeit (d2) stromauf vom Austauscher verschieden ist,
- das Messen zweier Werte Cd3in, Cd3out der Eigenschaft Cd in der dritten Flüssigkeit (d3) stromauf beziehungsweise stromab vom Austauscher, und
- das Berechnen wenigstens eines den Fortschritt der Behandlung kennzeichnenden Parameters (Cb, D, K, Kt/V) auf der Grundlage der Meßwerte der Eigenschaft Cd in der ersten (d1), der zweiten (d2) und der dritten (d3) Behandlungsflüssigkeit.

2. Verfahren nach Anspruch 1, dadurch gekennzeichnet, daß

- das Zeitintervall t2-ta zwischen dem Zeitpunkt ta, zu dem die zweite Flüssigkeit (d2) im Austauscher in Umlauf gebracht wird, und dem Zeitpunkt t2, zu dem der Wert Cd2out der Eigenschaft Cd in der zweiten Flüssigkeit stromab vom Austauscher gemessen wird, so gewählt wird, daß die Eigenschaft Cd stromab vom Austauscher zum Zeitpunkt t2 noch keinen stabilen Wert angenommen hat, und
- daß das Zeitintervall t3-tb zwischen dem Zeitpunkt tb, zu dem die dritte Flüssigkeit (d3) in dem Austauscher in Umlauf gebracht wird, und dem Zeitpunkt t3, zu dem der Wert Cd3out der Eigenschaft Cd in der dritten Flüssigkeit stromab vom Austauscher gemessen wird, so gewählt wird, daß die Eigenschaft Cd stromab vom Austauscher zum Zeitpunkt t3 noch keinen stabilen Wert angenommen hat.

3. Verfahren nach einem der Ansprüche 1 und 2, dadurch gekennzeichnet, daß stromauf vom Austauscher der Wert der Eigenschaft Cd in der ersten Flüssigkeit (d1) kleiner als der Wert der Eigenschaft Cd in der zweiten Flüssigkeit (d2) gewählt wird, der selber kleiner als der Wert der Eigenschaft Cd in der dritten Flüssigkeit (d3) gewählt wird.

4. Verfahren nach einem der Ansprüche 1 und 2, dadurch gekennzeichnet, daß stromauf vom Austauscher der Wert der Eigenschaft Cd in der ersten Flüssigkeit (d1) größer als der Wert der Eigenschaft Cd in der zweiten Flüssigkeit (d2) gewählt wird, der selber größer als der Wert der Eigenschaft Cd in der dritten Flüssigkeit (d3) gewählt wird.

5. Verfahren nach einem der Ansprüche 1 und 2, dadurch gekennzeichnet, daß stromauf vom Austauscher der Wert der Eigenschaft Cd in der ersten Flüssigkeit (d1) kleiner als der Wert der Eigenschaft Cd in der zweiten Flüssigkeit (d2) gewählt wird, der selber größer als der Wert der Eigenschaft Cd in der dritten Flüssigkeit (d3) gewählt wird.

6. Verfahren nach einem der Ansprüche 1 und 2, dadurch gekennzeichnet, daß stromauf vom Austauscher der Wert der Eigenschaft Cd in der ersten Flüssigkeit (d1) größer als der Wert der Eigenschaft Cd in der zweiten Flüssigkeit (d2) gewählt wird, der selber kleiner als der Wert der Eigenschaft Cd in der dritten Flüssigkeit (d3) gewählt wird.

7. Verfahren nach einem der Ansprüche 1 und 2, dadurch gekennzeichnet, daß als Funktion des Vorzeichens der Differenz Cdin1 - Cdout1 zwischen den Meßwerten der Eigenschaft Cd in der ersten Flüssigkeit (d1) stromauf und stromab vom Austauscher der Wert der Eigenschaft Cd in der zweiten Flüssigkeit (d2) stromauf vom Austauscher kleiner oder größer als der Wert der Eigenschaft Cd in der ersten Flüssigkeit (d1) stromauf vom Austauscher gewählt wird.

8. Verfahren nach einem der Ansprüche 1 und 2, dadurch gekennzeichnet, daß als Funktion des Vorzeichens der Differenz Cdin1 - Cdout1 zwischen den Meßwerten der Eigenschaft Cd in der ersten Flüssigkeit (d1) stromauf und stromab vom Austauscher und/oder als Funktion des Vorzeichens der Differenz Cdin2 - Cdout2 zwischen den Meßwerten der Eigenschaft Cd in der zweiten Flüssigkeit (d2) stromauf und stromab vom Austauscher der Wert der Eigenschaft Cd in der dritten Flüssigkeit (d3) stromauf vom Austauscher kleiner oder größer als der Wert der Eigenschaft Cd in der zweiten Flüssigkeit (d2) stromauf vom Austauscher gewählt wird.

9. Verfahren nach einem der Ansprüche 1 und 2, dadurch gekennzeichnet, daß der Wert der Eigenschaft Cd in der dritten Flüssigkeit (d3) stromauf vom Austauscher als Funktion eines Näherungswertes der Natriumkonzentration im Blut Cb' gewählt wird, der als Funktion der an den ersten und zweiten Dialyseflüssigkeiten (d1, d2) gemessenen Werten Cd1in, Cd1out, Cd2in, Cd2out berechnet wird.

10. Verfahren nach einem der Ansprüche 5 bis 8, dadurch gekennzeichnet, daß stromauf vom Austauscher der Wert der Eigenschaft Cd in der dritten Flüssigkeit (d3) im wesentlichen gleich dem Wert der Eigenschaft Cd in der ersten Flüssigkeit (d1) gewählt wird.

11. Verfahren nach einem der Ansprüche 2 bis 10, dadurch gekennzeichnet, daß die Zeitintervalle t2-ta und t3-tb im wesentlichen gleich gewählt werden.

12. Verfahren nach einem der Ansprüche 1 bis 11, dadurch gekennzeichnet, daß es außerdem den Schritt der Berechnung wenigstens eines zweiten Wertes des die Behandlung kennzeichnenden Parameters (Cb, D, K, Kt/V) aufweist.

13. Verfahren nach Anspruch 12, dadurch gekennzeichnet, daß es außerdem die folgenden Schritte aufweist:

- das Vergleichen von wenigstens zwei Werten des die Behandlung kennzeichnenden Parameters (Cb, D, K, Kt/V); und
- das Ausgeben eines Fehlersignals, falls die Werte des die Behandlung kennzeichnenden Parameters (Cb, D, K, Kt/V) nicht einer vorherbestimmten Regel folgen.

14. Verfahren nach einem der Ansprüche 1 bis 13, dadurch gekennzeichnet, daß die Eigenschaft der Behandlungsflüssigkeit (d), die gemessen wird, die Leitfähigkeit oder die Natriumkonzentration Cd ist und daß der die Behandlung kennzeichnende Parameter, der berechnet wird, die Konzentration des Natriums Cb im Blut stromauf vom Austauscher ist.

15. Verfahren nach den Ansprüchen 10, 11 und 14, dadurch gekennzeichnet, daß die Konzentration des Natriums Cb im Blut stromauf vom Austauscher nach folgender Formel berechnet wird:

$$Cb = \frac{Cd1out - (1 - A) \times Cd1in}{A}$$

wobei

$$A = 1 - \frac{(2 \times Cd1out - Cd2out - Cd3out)}{(2 \times Cd1in - Cd2in - Cd3in)}$$

16. Verfahren nach einem der Ansprüche 1 bis 13, dadurch gekennzeichnet, daß die Eigenschaft der Behandlungsflüssigkeit (d), die gemessen wird, die Leitfähigkeit oder die Natriumkonzentration Cd ist und daß der die Behandlung kennzeichnende Parameter, der berechnet wird, die Dialysance D ist.

17. Verfahren nach den Ansprüchen 10, 11 und 16, dadurch gekennzeichnet, daß die Dialysance D nach folgender Formel berechnet wird:

$$D = (Qd + Quf) \times A$$

wobei

Qd die Durchflußrate der Dialyseflüssigkeit ist,
Quf die Durchflußrate der Ultrafiltration ist und

$$A = 1 - \frac{(2 \times Cd1out - Cd2out - Cd3out)}{(2 \times Cd1in - Cd2in - Cd3in)}$$

18. Verfahren nach einem der Ansprüche 16 oder 17, dadurch gekennzeichnet, daß die Clearance K für einen Stoffwechselbestandteil des Blutes (Harnstoff, Kreatinin, etc.) aus der Dialysance D auf der Grundlage einer zuvor erstellten Zuordnungstabelle abgeleitet wird.

19. Verfahren nach Anspruch 18, dadurch gekennzeichnet, daß der die Behandlung kennzeichnende Parameter, der berechnet wird, die Dialysedosis Kt/V ist, wobei t die abgelaufene Behandlungszeit und V das Gesamtwasservolumen des Patienten ist.

20. Verfahren nach einem der Ansprüche 1 bis 19, dadurch gekennzeichnet, daß bei dem Schritt des Berechnens wenigstens eines Wertes wenigstens eines den Fortschritt der Behandlung kennzeichnenden Parameters (Cb, D, K, Kt/V), anstelle des Meßwertes Cd1in, Cd2in, Cd3in der Eigenschaft Cd in der ersten (d1), zweiten (d2) und dritten (d3) Behandlungsflüssigkeit, entsprechende Einstellwerte Cd1inREF, Cd2inREF, Cd3inREF verwendet werden, die vor dem Beginn jeder Behandlungssitzung einer die Herstellung der Behandlungsflüssigkeit steuernden Steuereinheit (26) zur Verfügung gestellt werden.

Fig. 1

Fig. 2